# EUROPEAN PATENT APPLICATION

(11) **EP 1 810 659 A1**
(43) Date of publication of application: **25.07.2007**
(21) Application number: 05292520.3
(22) Date of filing: 28.11.2005
(51) Int. Cl.: A61K 8/35, A61K 8/73, A61Q 19/04

(54) **Self-tanning compositions**

(71) Applicant: Johnson & Johnson Consumer France SAS, 92787 Issy les Moulineaux Cedex 9 (FR)
(72) Inventor: Brillouet, Anne Sophie, 27400 Louviers (FR); Baranger, Florence, 27100 Le andreuil France (FR); Harivel, Anne-Cécile, 94000 Creteil (FR)
(74) Representative: Metten, Karl-Heinz

(57) **Abstract**

Self-tanning compositions are provided. They comprise a self-tanning agent such as 1,3-dihydroxyacetone, optionally at least one dye, a modified starch gelling agent, and optionally a branched polysaccharide gelling agent. The compositions may be in the form of emulsions or foams and provide excellent stability of the self-tanning agent.

## Description

The present invention relates to a self-tanning composition comprising a self-tanning agent, preferably 1,3-dihydroxyacetone, a modified starch gelling agent, at least one dye, and, optionally, a branched polysaccharide gelling agent. The present invention further relates to a self-tanning composition comprising a self-tanning agent, preferably 1,3-dihydroxyacetone, a modified starch gelling agent, a branched polysaccharide gelling agent, and, optionally, at least one dye. The compositions may be in the form of emulsions or foams and provide excellent stability of the self-tanning agent.

### Background of the Invention

Because of the risks associated with sun tanning such as sunburn, many people use self-tanning compositions as a means to either achieve a tan without exposure to the sun, obtain a deeper tan with less exposure to the sun, or to extend the natural life of their suntan.

Self-tanning compositions typically contain 1,3-dihydroxylacetone as the self-tanning agent. A major disadvantage of this ingredient, however, is its lack of stability leading to discoloration and malodor in such compositions. Many self-tanning compositions also contain dyes to provide a visual aid to the user to prevent uneven administration on the skin surface and an immediate darkening effect on the skin. Most of these dyes, however, are nitrogen-based compounds and/or contain metal oxides, which are reactive with 1,3-dihydroxylacetone and therefore exacerbate its instability.

US Patent No. 6,214,322 to Castro et al. relates to the use of carmine instead of conventional dyes in self-tanning compositions. Carmine, advantageously, is not reactive with self-tanning agents such as 1,3-dihydroxylacetone.

A further need exists for self-tanning compositions containing self-tanning agents such as 1,3-dihydroxylacetone that are stable. The present invention relates to the use certain gelling agents in combination with self-tanning agents and optionally dyes to produce stable compositions.

### Summary of the Invention

The invention provides a sunless or self-tanning composition comprising: (a) a self-tanning agent having the formula: 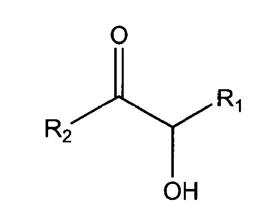 wherein R₁ is H, CH₂OH, CHOHCH₂OH, CH(OH)CH(=O), CH(NH₂)CH(=O), CH(OCH₃)CH(=O), or CH(NH-phenyl)CH(=O); and R₂ is H or CH₂OH; (b) a modified starch gelling agent; (c) at least one dye and, optionally, (d) a branched polysaccharide gelling agent.

The invention also provides a self-tanning composition comprising: (a) a self-tanning agent having the formula: 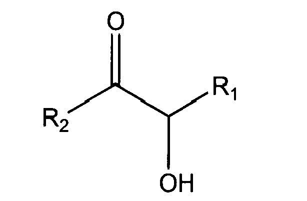 wherein R₁ is H, CH₂OH, CHOHCH₂OH, CH(OH)CH(=O), CH(NH₂)CH(=O), CH(OCH₃)CH(=O), or CH(NH-phenyl)CH(=O); and R₂ is H or CH₂OH; (b) a modified starch gelling agent; (d) a branched polysaccharide gelling agent; and, optionally, (c) at least one dye.

### Detailed Description of the Invention

The compositions of the invention comprise a self-tanning agent, that is, a chemical agent capable of producing or inducing the artificial tanning process of the skin by forming brown pigments in the skin, e.g., through the Maillard reaction reported in Bobin, et al., J. Soc. Cosmet. Chem., 35:265-72 (1984). In particular, the self-tanning agent is an α-hydroxy ketone or aldehyde of the formula: 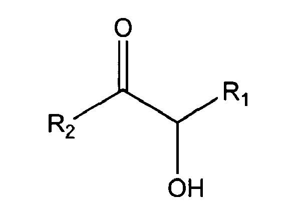 wherein R₁ is H, CH₂OH, CHOHCH₂OH, CH(OH)CH(=O), CH(NH₂)CH(=O), CH(OCH₃)CH(=O), or CH(NH-Phenyl)CH(=O); and R₂ is H or CH₂OH. Examples of such a compound include 1,3-dihydroxyacetone (i.e., dihydroxyacetone) and 1,3,4-trihydroxy-2-butanone (i.e., erythulose). In a preferred embodiment, the self-tanning agent is 1,3-dihydroxyacetone.

The amount of self-tanning agent in the compositions of the invention varies from about 0.1 to about 6, preferably from about 3 to about 5, weight percent based on the total weight of the composition.

The composition comprising a modified starch gelling agent and a branched polysaccharide gelling agent optionally contains at least one dye. Dyes are usually used to add color to the composition in order to help the user identify the area in which the composition has been applied and/or modify the color produced by the self-tanning agent in the composition.

Examples of dyes suitable for the compositions of the invention_include caramel, carmine, fluorescein derivatives, methoxsalen, trioxsalen, carbon black, azo dyes, anthraquinone dyes, blue azulenes, guajazulene, chamuzulene, erythrosin, bengal rose, phloxin, cyanosin, daphinin, eosin G, cosin 10B, Acid Red 51, Red Dye 4, Red Dye 40, Blue Dye 1, and Yellow Dye 5, or mixtures thereof. Other dyes are listed on page 1628-30 of the International Cosmetic Ingredient Dictionary and Handbook, eds. Wenninger and McEwen (The Cosmetic, Toiletry, and Fragrance Assoc., Washington, D.C., 7th Edition, 1997) (hereinafter "ICT Handbook"), the contents of which are incorporated herein by reference.

When used, the amount of dye in the composition may vary from about 0.0001 to about 0.1, preferably about 0.0025 to about 0.025, weight percent based on the total weight of the composition.

The compositions of the invention further contain a modified starch gelling agent, for example a crosslinked starch, modified corn starch, hydroxypropylated starch, or a starch phosphate ester. In one embodiment, the modified starch gelling agent is a nonionic polymer. In another embodiment, the modified starch gelling agent has shear thinning properties. In a further embodiment, the modified starch gelling agent is both nonionic and shear thinning. Preferably, the modified starch gelling agent is hydroxypropyl starch phosphate. Hydroxypropyl starch phosphate is commercially available under the name STRUCTURE XL from National Starch and Chemical.

The composition of the invention comprising the self-tanning agent, a modified starch gelling agent, and at least one dye is preferably used in the form of an emulsion.

The amount of modified starch gelling agent in the compositions of the invention varies from about 0.1 to about 6, preferably from about 0.5 to about 3, weight percent based on the total weight of the composition.

According to another embodiment of the invention the composition contains in addition to a self-tanning agent and a modified starch gelling agent also a branched polysaccharide gelling agent. This composition is preferably used in the form of a foam. For example, the branched polysaccharide gelling agent may be selected from xanthan gum and succinoglycan. Preferably, the branched polysaccharide gelling agent is succinoglycan. Succinoglycan is commercially available under the name RHEOZAN SH from Rhodia.

The amount of branched polysaccharide gelling agent in the composition varies from about 0.01 to about 1, preferably from about 0.15 to about 0.20, weight percent based on the total weight of the composition.

The compositions of the invention may contain additional gelling agents, e.g. based on polyacrylates, such as polyacrylate 13/polyisobutylate/polysorbate 20 blend. Polyacrylate 13/polyisobutylate/polysorbate 20 blend is commercially available under the name SEPIPLUS 400 from Seppic.

The compositions of the invention may contain various other optional ingredients known in the art, such as acidifying agents, alkalizing agents, aerosol propellants, antimicrobial agents, antioxidants, buffering agents, chelating agents, coloring additives, dermotologicaly active agents, dispersing agents, emollients, emulsifying agents, humectants, fragrances, preservatives, sugars, sunscreen agents, surfactants, suspending agents, thickening agents, and vehicles. Examples of these ingredients are listed below as well as in the ICT Handbook.

Acidifying and alkalizing agents can be added to obtain the desired pH of the composition. Examples of acidifying agents included citric acid, lactic acid, glycolic acid, acetic acid, glacial acetic acid, malic acid, and proprionic acid. Examples of alkalizing agent include edetol, potassium carbonate, potassium hydroxide, sodium borate, sodium carbonate, sodium citrate, sodium lactate, sodium glycolate, and sodium hydroxide. Other acidifying and alkalizing agents are listed on page 1653 of the ICT handbook.

Aerosol propellants can be used when the composition is to be administered as an aerosol under pressure. Examples of aerosol propellants include halogenated hydrocarbons such as dichlorodifluoromethane, dichlorotetrafluoroethane, and trichloromonfluoromethane, nitrogen, and volatile hydrocarbons such as butane, propoane, isobutane, or mixtures thereof. Other aerosol propellants are listed on page 1655 of the ICT handbook.

Antimicrobial agents can be used when the area that the composition is to be applied is prone to microbial infection, e.g., by bacteria, fungal, or protozoa. Examples of such agents include benzyl alcohol, chlorobutanol, phenylethyl alcohol, phenylmercuric acetate, potassium sorbate, and sorbic acid, benzoic acid, butylparaben, ethylparaben, methylparaben, propylparaben, and sodium benzoate. Other antimicrobial agents are listed on page 1612 of the ICT handbook.

Antioxidants can be used to protect ingredients of the composition from oxidizing agents that are included within or come in contact with the composition. Examples of antioxidants include water soluble antioxidants such as ascorbic acid, sodium sulfite, metabisulfite, sodium miosulfite, sodium formaldehyde, sulfoxylate, isoascorbic acid, isoascorbic acid, cysteine hydrochloride, 1,4-diazobicyclo-(2,2,2)-octane, and mixtures thereof. Examples of oil-soluble antioxidants include ascorbyl palmitate, butytlated hydroxyanisole, butylated hydroxytoluene, potassium propyl gallate, octyl gallate, dodecyl gallate, phenyl-α-napthyl-amine, and tocopherols such as α-tocopherol. Other agents are listed on pages 1612-13 of the ICT Handbook.

Buffering agents can be used to maintain an established pH of the composition. Examples of buffering agents included calcium acetate, potassium metaphosphate, potassium phosphate monobasic, and tataric acid. Other buffering agents are listed on page 1612 of the ICT handbook.

Chelating agents can be used to maintain the ionic strength of the composition and/or bind to destructive compounds and metals that are included within or come in contact with the composition. Examples of chelating agents included edatate dipotassium, edetate disodium, edetic acid, and ethylenediamine tetracetic acid (EDTA) and its salts (e.g., tetrasodium EDTA). Other chelating agents are listed on page 1626 of the ICT handbook.

Dermotalogically active agents include agents for treating wound healing, inflammation, acne, psoriasis, cutaneous aging, skin cancer, impetigo, herpes, chickenpox, dermatitis, pain, itching, skin irritation. Examples of such dermatalogically active agents include hydrocortisone, dexamethesone, panthenol, phenol, tetracycline hydrochloride, yeast, hexylresorcinol, lamin, kinetin, betamethasone, triamcinolone, fluocinolone, methylprednisolone, retnoids such as retinol and retinoic acid, dapsone, sulfasalazine, resorcinol, salicylic acid, benzoyl peroxide, erythromycin-benzoyl peroxide, erythromycin, clindamycin, mupirocin, griseofulvin, azoles such as miconazole, econozole, itraconazole, fluconazole, and ketoconazole, ciclopirox, allylamines such as naftifine and terfinafine, acyclovir, famciclovir, valacyclovir, benzocaine, lidocaine, dibucaine, pramoxine hydrochloride, methyl salicylate, camphor, menthol, resocinol, and vitamins such as tocopherol, and tocopherol acetate.

Examples of dispersing and suspending agents include poligeenan, magnesium aluminum silicate xanthum gum, and silicon dioxide. Other dispersing or suspending agents are listed on page 1612 of the ICT handbook.

Emollients are agents that soften and smooth the skin. Examples of emollients include hydrocarbon oils and waxes such as mineral oil, petrolatum, microcrystaline wax, polyethylene, triglyceride esters such as those of castor oil, cocoa butter, safflower oil, cottonseed oil, corn oil, olive oil, cod liver oil, almond oil, avocado oil, palm oil, sesame oil, squalene, and soybean oil, acetylated monoglycerides, ethoxylated glycerides, fatty acids, alkyl esters of fatty acids, alkenyl esters of fatty acids, fatty alcohols, fatty alcohol ethers, ether-esters, lanolin and derivatives of lanolin, polyhydric alcohol esters, wax esters such as beeswax, vegetable waxes, phospholidds, and sterols. Other emollients are listed on pages 1656-61 of the ICT handbook.

Emulsifying agents can be used for preparing the oil-in-water emulsions of the present invention. Examples of emulsifying agents include Arlacel 165 and methyl gluceth sesquisterate, fatty alcohols, fatty alcohols and alkyl phenols condensed with ethylene oxide. Other emulsifiers are listed on pages 1679-87 of the ICT Handbook. Emulsion stabilizers are listed on pages 1634-35 of the ICT Handbook.

Humectants are agents that typically promote the retention of moisture, e.g., moisturizers. Examples of humectants include sorbitol, glycerin, glycereth 5 lactate, glycereth 7 triacetate, glycereth 7 diisononoate, hexanetriol, glycols such as methylpropanediol, 1,2-pentanediol, hexylene glycol, and propylene glycol, alkoxylated glucose, D-panthenol and derivatives thereof, and hyaluronic acid. Other humectants are listed on pages 1661-62 of the ICT Handbook.

Examples of fragrances include peppermint, rose oil, rose water, aloe vera, clove oil, menthol, camphor, eucalyptus oil, and other plant extracts. To eliminate certain odors from compositions, masking agents may be used. An example of a masking agent includes ethylene brassylate. Other fragrances and masking agents are listed on pages 1639-40 of the ICT Handbook.

Preservatives can be used to protect the composition from degradation. Examples of preservatives include phenoxyethanol, methylparaben, benzalkonium chloride, benzethonium chloride, propyl paraben, benzoic aicd, benzyl alcohol, and mixtures thereof such as liquipar oil. Other preservatives are listed on pages 1654-55 of the ICT Handbook.

Sugars can be used to improve the results obatined by the self-tanning agents. Examples of sugars include monosaccharides, disaccharides, and polysccharides such as glucose, xylose, fructose, reose, ribose, pentose, arabinose, allose, tallose, altrose, mannose, galactose, lactose, sucrose, erythrose, glyceraldehyde, or any combination thereof.

Sunscreen agents are agents typically used to block or reduce the amount of ultraviolet radiation impinging on the skin (e.g., by absorption, scattering, and reflection of the ultraviolet radiation). Segarin, et al., Cosmetics Science and Technology, Chapter VIII, pages 189, et seq. discloses numerous examples of sunscreen agents. Examples of sunscreen agents include both organic compounds and their salts such as, butylmethoxydibenzoyl methane, diethyl hexyl butamido triazone, diethyl amino hydroxybenzoyl hexyl benzoate, ethylhexyl triazone, bis-ethylhexyloxyphenol methoxyphenyl triazine, methylene bis-benzotriazolyl tetramethylbutyl phenol, phenyl benzymidazole sulfonic acid, ethylhexyl salicylate, benzophenone-3 homosalate, octocrylate, avobenzone, and menthyl anthranilate, as well as inorganic particulate materials such as zinc oxide, silica, iron oxide, titanium dioxide, and 2-ethyl-hexyl-p-methoxycinnamate. Other agents are listed on page 1672 of the ICT Handbook. Generally, the composition may contain from about 1% to about 50%, by weight, of sunscreen agent(s). The exact amounts will vary depending on the sunscreen used and the desired sun-protection factor (SPF).

Surfactants are agents typically used to stabilize multi-component compositions, e.g., used as wetting agents, antifoam agents, emulsifiers, dispersing agents, and penetrates. Examples of surfactants include lapyrium chloride, laureth 4, laureth 9, monoethanolamine, nonoxynol 4, nonoxynol 9, nonoxynol 10, nonoxynol 15, nonoxynol 30, poloxalene, polyoxyl 8, 40, and 50 stearate, polysorbate 20, polysorbate 40, polysorbate 60, polysorbate 65, polysorbate 80, and polysorbate 85, sodium lauryl sulfate, sorbitan, decyl polyglucoside, PPG-5-ceteth-20 and its derivatives. Other surfactants are listed on pages 1672-90 of the ICT Handbook.

Vehicles are often referred to as the base for the cosmetically acceptable carrier, e.g., a fluid that is capable of delivering the other components of the composition to the skin with acceptable absorption of those components into the skin. Examples of vehicles include water, e.g., deionized water, oil-in-water emulsions, e.g., where the continuous water phase contains the water soluble agents and the discontinuous oil phase contains the oil soluble agents, and water-in-oil emulsions.

The composition may be in a number of different delivery forms, e.g., a spray, foam, mist, aerosol, semi-solid cream, liquid such as a solution, emulsion, suspension, lotion, gel, solid such as a powder, adherent stick, flexible mask, or self-hardening liquid or gel, or other suitable forms intended to be applied to the hair, skin, or nails. Water-in-oil emulsions (e.g., ratio of about 10:1 1 to about 1:100 such as about 1:1 to about 1:10) and oil-in-water emulsions (e.g., ratio of about 10:1 1 to about 1:100 such as about 1:1 to about 1:10) are typically used in preparing emulsions, lotions and creams.

The viscosity of the composition may be different dependent on the type of formulation being prepared, e.g., a liquid formulation will have a lower viscosity than a gel formulation. Typically, the viscosity of cream formulations ranges from 5,000 to 150,000 cps. Bulking agents may be used to increase the viscosity of the composition. An example of a bulking agent is talc. Other bulking agents are listed on page 1625-26 of the ICT Handbook. Other viscosity increasing agents are listed on pages 1693-97 of the ICT Handbook. Viscosity decreasing agents are listed on pages 1692-92 of the ICT Handbook.

In one embodiment, the composition comprises an emulsion comprising the self-tanning agent, a dye and a modified starch gelling agent. Preferably, such composition comprises about 3 to about 5 weight percent 1,3-dihydroxyacetone, about 0.002 to about 0.003 weight percent Blue Dye 1, about 0.02 to about 0.03 weight percent Red Dye 40, about 0.02 to about 0.04 weight percent Yellow Dye 5, and 1 to about 4 weight percent hydroxypropyl starch phosphate.

In another embodiment, the composition comprises a foam comprising the self-tanning agent, a modified starch gelling agent and a branched polysaccharide gelling agent. Preferably, such composition comprises about 3 to about 5 weight percent 1,3-dihydroxyacetone, about 0.1 to about 1 weight percent hydroxypropyl starch phosphate, and 0.15 to about 0.20 weight percent starch succinoglycan.

The following non-limiting examples further illustrate the invention.

### Example 1 - Lotion

A self-tanning composition according to the invention was prepared in the form of an emulsion (lotion). It contained the following ingredients:

| Ingredient | Weight % |
|---|---|
| Aqua | Qsp 100 |
| Hydroxypropyl Starch Phosphate | 1,000 |
| Succinoglycan | 0,150 |
| Glycerin | 1,000 |
| Polyacrylate 13/polyisobutene/ Polysorbate20 | 0,300 |
| Methylparaben | 0,200 |
| Phenoxyethanol | 0,700 |
| Sorbitol 70%/ Aqua 30% | 3,000 |
| Arachidyl Behenyl Alcohols/ Arachidylglucoside | 3,000 |
| PEG-100 Stearate 50%/Glyceryl Stearate 50% | 1,500 |
| Propylparaben | 0,100 |
| Ethylhexyl Stearate | 10,000 |
| Cetyl Alcohol | 0,500 |
| Tocopherol 85%/Hydrogenated Palm Glycerides Citrate 15% | 0,150 |
| Cyclohexasiloxane 91 %/Cyxlopentasiloxane 4% | 2,000 |
| Aqua | 10,000 |
| Dihydroxyacetone | 5,000 |
| Aqua | 1,000 |
| Acide citrique | 0,026 |
| Aqua | 1,500 |
| Yellow Dye 5 | 0,025 |
| Red Dye 40 | 0,025 |
| Blue Dye 1 1 %/ Aqua 99% | 0,276 |
| Parfum | 0,300 |

The stability of the composition was evaluated in terms of color and pH. Measurements were taken after 6 weeks at 50° C and after 13 weeks at 40° C. Color was evaluated by visual assessment of three assessors using a scale of 0-10, 10 being the initial color and 0 being damaged color.

A comparative composition was also tested for color stability and pH. The comparative composition contained the following ingredients:

| Ingredient | Weight % |
|---|---|
| Aqua | QSP 100% |
| Acrylates/C10-30 Alkyl acrylate crosspolymer | 0.1 |
| Xanthan gum | 0.2 |
| Butylene glycol | 3 |
| Glycerin | 3 |
| Hexylene glycol | 3 |
| Phenoxyethanol | 0.6 |
| Methyl Paraben | 0.2 |
| Sodium disulfite | 0.2 |
| Disodium EDTA | 0.1 |
| Citric Acid | 0.13 |
| Isononyl Isononoate | 2 |
| Dimethicone | 2 |
| Bis-PEG/PPG-16/PPG-16/16 Dimethicone >50%/Caprylic/Capric Triglyceride 10-25% | 2 |
| Cetyl alcohol | 0.75 |
| Aqua | 10 |
| Dihydroxyacetone | 5 |
| Tocopherol Acetate | 1 |
| BHT | 0.1 |
| Nylon-12 | 0.5 |
| Sodium Acrylate/Sodium acryloyldimethyl taurate copolymer 25%/Isohexadecane 20%/Polysorbate 80 7%/Aqua 48% | 4 |
| Yellow Dye 5 | 0.02904 |
| Red Dye 4 | 0.02852 |
| Blue Dye 1 | 0.0024 |
| Parfum | 0.3 |

The results are shown in Tables 1 and 2.

**TABLE 1: Color Assessment**

| | **T₀** | **6W50°C** | **13W40°C** |
|---|---|---|---|
| **Comparative composition (lotion)** | **10** | **2** | **4** |
| **Composition according to the invention (lotion)** | **10** | **5** | **7** |

**TABLE 2: pH**

| | pH at T₀ | pH at 6 weeks 50°C | PH at 13 weeks 40°C | PERCENTAGE OF pH decrease after 6W50°C | PERCENTAGE OF pH decrease after 13W40°C |
|---|---|---|---|---|---|
| Comparative composition (lotion) | 4,45 | 2,93 | 3,1 | 34,16 | 30,34 |
| Composition according to the invention (lotion) | 4,19 | 3,2 | 3,38 | 22,67 | 19,33 |

### Example 2 - Foam

A self-tanning composition according to the invention was prepared in the form of a foam. It contained the following ingredients:

| Ingredient | Weight % |
|---|---|
| Aqua | Qsp 100 |
| Dihydroxyacetone | 5,00 |
| Pentylene Glycol | 3,00 |
| Methyl Gluceth-20 | 1,00 |
| Glycerin | 1,00 |
| Decyl Polyglucose | 1,00 |
| PPG-5-Ceteth-20 | 1,00 |
| Methyl Propanediol | 0,50 |
| Succinoglycan | 0,20 |
| Hydroxypropyl Starch phosphate; aqua | 0,50 |
| Phenoxyethanol, methyl paraben, propyl paraben, ethyl paraben | 0,60 |
| Citric Acid | 0,05 |
| Parfum | 0,20 |

The stability of 1,3-dihydroxyacetone (DHA) was measured after 4 weeks at 40°C, 6 weeks at 50° C, and 13 weeks at 40° C using HPLC.

A comparative composition was also tested for DHA stability in the same manner. The comparative composition contained the following ingredients:

| Ingredient | Weight % |
|---|---|
| Aqua | Qsp 100 |
| Dihydroxyacetone | 5,00 |
| Pentylene Glycol | 3,00 |
| Methyl Gluceth-20 | 1,00 |
| Glycerin | 1,00 |
| Decyl Polyglucose | 1,00 |
| PPG-5-Ceteth-20 | 1,00 |
| Erythrulose | 0,50 |
| Methyl Propanediol | 0,50 |
| Cetyl Hydroxyethylcellulose | 0,50 |
| Sodium Citrate | 0,35 |
| Phenoxyethanol, methyl paraben, propyl paraben, butylparaben, ethylparaben, isobutylparaben | 0,60 |
| Citric Acid | 0,20 |
| Parfum | 0,20 |

The results are shown in Table 3 below.

**TABLE 3: DHA Loss**

| | Comparative Composition (foam) | Composition according to the invention (foam) |
|---|---|---|
| Amount of DHA at T₀ | 4,9 | 4,9 |
| Amount of DHA after 4 weeks at 40°C | 3,2 | 4,7 |
| Amount of DHA after 6 weeks at 50°C | 2 | 3,75 |
| Amount of DHA after 13 weeks at 40°C | 1,3 | 4 |
| PERCENTAGE OF DHA LOSS after 4W40°C | 34,7 | 3,9 |
| PERCENTAGE OF DHA LOSS after 6W50°C | 59,18 | 23,47 |
| PERCENTAGE OF DHA LOSS after 13W40°C | 73,47 | 18,37 |

## Claims

1. A self-tanning composition comprising:
(a) a self-tanning agent having the formula: 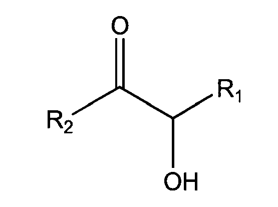 wherein R₁ is H, CH₂OH, CHOHCH₂OH, CH(OH)CH(=O), CH(NH₂)CH(=O), CH(OCH₃)CH(=O), or CH(NH-phenyl)CH(=O); R₂ is H or CH₂OH;
(b) a modified starch gelling agent; and
(c) at least one dye.

2. The composition of claim 1, further comprising (d) a branched polysaccharide gelling agent.

3. The composition according to claim 1, wherein said composition is in the form of a foam or in particular in the form of an emulsion.

4. A self-tanning composition comprising:
(a) a self-tanning agent having the formula: 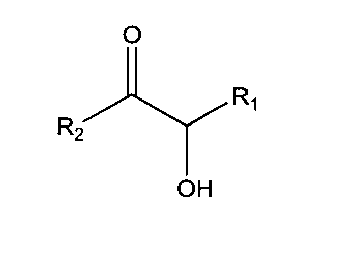 wherein R₁ is H, CH₂OH, CHOHCH₂OH, CH(OH)CH(=O), CH(NH₂)CH(=O), CH(OCH₃)CH(=O), or CH(NH-phenyl)CH(=O); R₂ is H or CH₂OH;
(b) a modified starch gelling agent; and
(d) a branched polysaccharide gelling agent.

5. The composition of claim 4, further comprising (c) at least one dye.

6. The composition according to claim 4, wherein said composition is in the form of an emulsion or in particular in the form of a foam.

7. The composition according to any of the preceding claims, wherein the modified starch gelling agent comprises crosslinked starch, modified corn starch, hydroxypropylated starch and/or a starch phosphate ester.

8. The composition of any of claims 2 to 7, wherein the branched polysaccharide gelling agent is succinoglycan.

9. The composition according to any of the preceding claims, wherein the self-tanning agent is 1,3-dihydroxyacetone.

10. The composition according to any of the preceding claims, wherein the dye is selected from Red Dye 4, Red Dye 40, Blue Dye 1, Yellow Dye 5, caramel, carmine, fluorescein derivatives, methoxsalen, trioxsalen, carbon black, azo dyes, anthraquinone dyes, blue azulenes, guajazulene, chamuzulene, erythrosin, bengal rose, phloxin, cyanosin, daphinin, eosin G, cosin 10B, and Acid Red 51.

11. The composition according to any of the preceding claims, wherein the modified starch gelling agent is hydroxypropyl starch phosphate.

12. The composition according to any of the preceding claims, further comprising as an additional gelling agent a polyacrylate 13/polyisobutylate/polysorbate 20 blend.

13. The composition according to any of the preceding claims comprising about 0.1 to about 6 weight percent self-tanning agent (a).

14. The composition according to any of the preceding claims comprising about 0.0001 to about 0.1 weight percent dye (c).

15. The composition according to any of the preceding claims comprising about 0.1 to about 6 weight percent modified starch gelling agent (b).

16. The composition according to any of claims 12 to 15 comprising about 0.1 to about 6 weight percent modified starch gelling agent, about 0.01 to about 1 weight percent succinoglycan and about 0.1 to about 2 weight percent polyacrylate 13/polyisobutylate/polysorbate 20 blend.

17. The composition according to any of claims 2 to 16 comprising about 0.01 to about 1 weight percent branched polysaccharide gelling agent (d).

18. The composition according to any of the preceding claims further comprising a potato starch gelling agent.

19. Use of a self-tanning agent having the formula 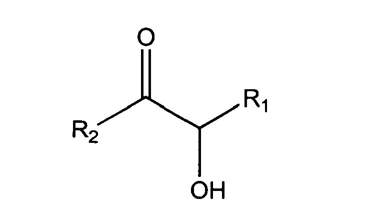 wherein R₁ is H, CH₂OH, CHOHCH₂OH, CH(OH)CH(=O), CH(NH₂)CH(=O), CH(OCH₃)CH(=O), or CH(NH-phenyl)CH(=O); and R₂ is H or CH₂OH; a modified starch gelling agent, at least one dye, and, optionally, a branched polysaccharide gelling agent for the preparation of a self-tanning composition.

20. Use of a self-tanning agent having the formula 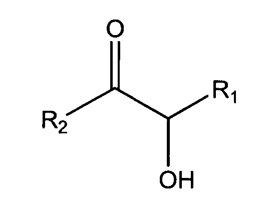 wherein R₁ is H, CH₂OH, CHOHCH₂OH, CH(OH)CH(=O), CH(NH₂)CH(=O), CH(OCH₃)CH(=O), or CH(NH-phenyl)CH(=O); and R₂ is H or CH₂OH; a modified starch gelling agent, a branched polysaccharide gelling agent, and, optionally, at least one dye for the preparation of a self-tanning composition.

21. Use of a self-tanning agent having the formula 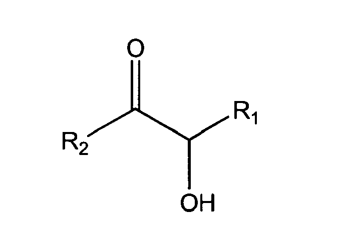 wherein R₁ is H, CH₂OH, CHOHCH₂OH, CH(OH)CH(=O), CH(NH₂)CH(=O), CH(OCH₃)CH(=O), or CH(NH-phenyl)CH(=O); and R₂ is H or CH₂OH; of a modified starch gelling agent, of at least one dye, and, optionally, of a branched polysaccharide gelling agent as a self-tanning composition.

22. Use of a self-tanning agent having the formula 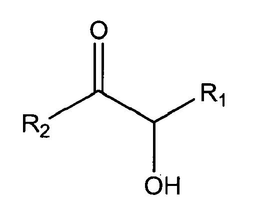 wherein R₁ is H, CH₂OH, CHOHCH₂OH, CH(OH)CH(=O), CH(NH₂)CH(=O), CH(OCH₃)CH(=O), or CH(NH-phenyl)CH(=O); and R₂ is H or CH₂OH; a modified starch gelling agent, a branched polysaccharide gelling agent, and, optionally, at least one dye as a self-tanning composition.
